# EUROPÄISCHE PATENTSCHRIFT

(11) **EP 1 020 197 B1**
(45) Veröffentlichungstag und Bekanntmachung des Hinweises auf die Patenterteilung: **12.03.2003**
(21) Anmeldenummer: 99126270.0
(22) Anmeldetag: 31.12.1999
(51) Int. Cl.: A61L 15/38

(54) **Verbandmaterial mit dem Sekret von Fliegenlarven**
Bandages containing maggot secretions
Bandages contenant la sécrétion des asticots

(30) Priorität: 14.01.1999 DE 19901134
(43) Veröffentlichungstag der Anmeldung: 19.07.2000
(60) Teilanmeldung: 02014517.3
(73) Patentinhaber: Fleischmann, Wilhelm, Dr. med., 74321 Bietigheim-Bissingen (DE)
(72) Erfinder: Fleischmann, Wilhelm, Dr. med., 74321 Bietigheim-Bissingen (DE)
(74) Vertreter: Patentanwälte Westphal, Mussgnug & Partner

(56) Entgegenhaltungen:
- EP-A- 0 236 610
- SHERMAN R A: "A new dressing design for use with maggot therapy" PLASTIC AND RECONSTRUCTIVE SURGERY, Bd. 100, 1997, Seiten 451-456, XP000905285
- PRETE P E: "Growth effects of Phaenicia Sericata larval extracts on fibroblasts: Mechanism for wound healing by maggot therapy" LIFE SCIENCES, Bd. 60, Nr. 8, 1997, Seiten 505-510, XP000905619
- FINE A ET AL: "Maggot Therapy. Technique and Clinical Application" JOURNAL OF BONE AND JOINT SURGERY, Bd. 16A, 1934, Seiten 572-582, XP000905606

## Beschreibung

Die Erfindung betrifft ein Verbandsmaterial zur Behandlung von Wunden.

Zur Behandlung von Wundinfektionen und Wunden, die abgestorbenes Gewebe enthalten, z.B. zur Behandlung der diabetischen Gangrän werden Fliegenlarven eingesetzt, insbesondere Larven der Fliegengattung Lucilia (Schmeißfliege) und insbesondere der Lucilia sericata, (Prete P.E. "Growth effects of phaenicia sericata larval extracts on fibroblasts: Mechanism for wound healing by maggot therapy", Life Sciences, Vol.60, No.8, 1997, Seiten 505-510). Die Fliegenlarven (Maden) werden für eine gewisse Zeit, z.B. etwa drei Tage, in die schwer therapierbare Wunde eingesetzt. Es hat sich gezeigt, daß die Maden in dieser Zeit abgestorbenes Gewebe in der Wunde entfernen, bakterielle Entzündungen beseitigen und die Wundheilung stimulieren.

Bei der bisher angewendeten Methode der Madenbehandlung (Sherman R.A. "A new dressing design for use with maggot therapy", Plastic and Reconstructive Surgery, Vol.100, 1997, Seiten 451-456) wird zunächst nach Reinigung der Wunde der Wundrand mit einem Klebestreifen abgeklebt. Die Maden werden auf ein feinmaschiges Netz aufgebracht, das dann gewendet und auf die Klebestreifen so aufgeklebt wird, daß die Maden sich zwischen dem Netz und der Wundoberfläche befinden. Das Netz wirkt so als luftdurchlässiger Käfig, der die Maden in der Wunde hält. Nach der Einwirkungszeit von etwa drei Tagen wird das Netz abgezogen und die Maden werden aus der Wunde entfernt.

Bei dieser Methode können verschiedene Probleme auftreten. Die Klebeverbindung des Netzes mit dem Wundrand ist nicht absolut zuverlässig. Löst sich der Kleberand, so ist nicht mit Sicherheit auszuschließen, daß Maden entweichen und sich verpuppen, so daß sich Schmeißfliegen entwickeln können. Das Entfernen der Maden aus der Wunde nach Abschluß der Behandlung ist mühsam und insbesondere auch für den Patienten unästhetisch. Bei größeren Wunden kann nicht sichergestellt werden, daß die Maden insbesondere dort aktiv werden, wo die stärkste therapeutische Wirkung erzielt werden soll.

Der Erfindung liegt die Aufgabe zugrunde, ein Verbandsmaterial zur Behandlung von Wunden zur Verfügung zu stellen, welches diese Nachteile der bekannten Madenbehandlung beseitigt oder verringert.

Diese Aufgabe wird erfindungsgemäß gelöst durch ein Verbandsmaterial mit den Merkmalen des Anspruchs 1.

Vorteilhafte Ausführungen der Erfindung sind in den Unteransprüchen angegeben.

Die Erfindung basiert auf der Erkenntnis, dass die wundtherapeutische Wirkung der Maden insbesondere durch das von den Maden abgesonderte Sekret hervorgerufen wird. Durch dieses Sekret, insbesondere ein Verdauungssekret wird abgestorbenes Gewebe verflüssigt, so daß es von den Maden als Nahrung aufgenommen werden kann. Das Sekret hat zusätzlich eine stark bakterienabtötende Wirkung und fördert die Wundheilung.

Der wesentliche Gedanke der Erfindung besteht darin, die Maden nicht frei in die Wunde einzusetzen, sondern eine Wundauflage zu verwenden, die das für den therapeutischen Erfolg wichtige Sekret der Fliegenlarven enthält. Die Wundauflage kann gezielt dort plaziert werden, wo das Sekret seine therapeutische Wirkung entfalten soll. Auch bei größeren Wunden kann auf diese Weise sichergestellt werden, daß die gesamte Wundfläche in gleicher Weise mit dem Sekret in Kontakt kommt. Ebenso können bestimmte Wundbereiche gezielt mit einer höheren Dosis und Konzentration des Sekrets behandelt werden.

Die Wundauflage kann in guten Kontakt mit der Wundoberfläche gebracht werden. Hierzu kann ggf. auch eine Einlage verwendet werden, die die Wundauflage, an der Wundoberfläche anliegend hält. Der gute Flächenkontakt der Wundauflage mit der Wundoberfläche gewährleistet, daß das Sekret im gesamten Bereich der Wundauflage gut wirksam wird. Die Wundauflage kann nach Beendigung der Einwirkungsdauer einfach, problemlos und rückstandsfrei aus der Wunde entfernt werden.

Die Wundauflage weist einen die Fliegenlarven einschließenden Beutel aus einem feinmaschigen netzartigen Material vorzugsweise einem Textilmaterial auf. Ein oder mehrere Beutel, welche die Maden enthalten, werden in die Wunde eingelegt. Die in dem Beutel eingeschlossenen Maden haben keine Möglichkeit, zu entweichen oder auch in andere Wundbereiche abzuwandern. Dadurch ist nicht nur sichergestellt, daß die Maden nicht aus der Wunde entweichen können, sondern es ist insbesondere auch sichergestellt, daß die Maden ihre therapeutische Wirkung in dem Wundbereich entfalten, auf welchen der Beutel aufgelegt ist. Es ist möglich, gezielt eine verstärkte therapeutische Wirkung in bestimmten Wundbereichen zu erzeugen. Ebenso kann durch das Aufbringen mehrerer kleinflächiger Beutel oder durch einen großflächigen Beutel, der in kleinere geschlossene Kammern unterteilt ist, sichergestellt werden, daß die Maden gleichmäßig über die gesamte Wundfläche verteilt sind und sich nicht in unerwünschter Weise in bestimmten Wundbereichen ansammeln, in welchen möglicherweise ihre therapeutische Wirkung weniger erforderlich ist. Schließlich ist durch das Einschließen der Maden in den Beuteln ein schnelles, einfaches und zuverlässiges Entfernen der Maden aus der Wunde möglich. Da der Patient die in dem Beutel eingeschlossenen Maden nicht sieht, ist auch das ästhetische und psychologische Problem der Madenbehandlung erheblich verringert.

Das Material, aus welchem die Beutel gefertigt sind, ist so feinmaschig, daß die Maden zuverlässig in dem Beutel eingeschlossen sind. Die Maschenweite des Materials, d. h. seine Porengröße ist so groß, daß ein ungehinderter Flüssigkeitsaustausch des Verdauungssekrets der Maden und des aufgelösten und verflüssigten nekrotischen Gewebes möglich ist. Weiter ist das Textilmaterial der Beutel ausreichend luftdurchlässig, um das Überleben der Maden sicherzustellen.

Die steril gezüchteten Fliegenlarven werden in die Beutel eingesetzt. Es ist auch möglich, die Fliegeneier in die Beutel einzubringen, so daß die Maden bereits in dem Beutel schlüpfen. Um die Maden bis zu ihrem Einbringen in die Wunde am Leben zu halten, ist es ggf. zweckmäßig oder notwendig, madenspezifische Nahrungspartikel zusammen mit den Maden in die Beutel einzubringen oder die Beutel damit zu imprägnieren. Da sich die Maden nur von totem Gewebe ernähren, kann eine solche Zugabe von Nahrungsstoffen auch dann von Bedeutung sein, wenn der Wundbereich, in welchem der Beutel aufgebracht wird, nicht oder nicht mehr ausreichend nekrotisches Gewebe enthält.

Für die praktische Verwendung der Wundauflage kann es von Vorteil sein, großflächigere Beutel zur Verfügung zu stellen, die dann bei der Applikation entsprechend der Wunde zugeschnitten werden. Hierzu kann der Beutel durch geeignete Mittel, z.B. durch Kunststoffklammern oder Klebemittel, unterteilt werden, so daß eine Durchtrennung möglich ist und nach der Durchtrennung die Teilbeutel an der Trennlinie verschlossen sind. Vorteilhaft kann es auch sein, eine großflächige z.B. in Bahnen zur Verfügung stehende Wundauflage zu verwenden, die in kleine geschlossene Kammern unterteilt ist, welche jeweils einige wenige Maden aufnehmen. Diese Wundauflage kann aufgrund der Rasterteilung in Kammern sehr einfach bedarfsgerecht zugeschnitten werden. Die Unterteilung in Kammern stellt sicher, daß die Maden gleichmäßig über die gesamte Fläche der Wundauflage verteilt gehalten werden und ihr Sekret gleichmäßig abgeben.

Um insbesondere bei größeren und tieferen Wunden die Wundauflage in guten Kontakt mit der Wundoberfläche zu bringen, ist es zweckmäßig, wenn das Verbandsmaterial zusätzlich eine Einlage aufweist, die auf die Wundauflage aufgelegt wird und durch die äußere Wundabdeckung gehalten wird. Durch die Wundabdeckung wird die Einlage auf die Wundauflage gedrückt, wodurch wiederum die Wundauflage in guten Kontakt mit der Wundoberfläche gebracht wird. Die Einlage besteht vorzugsweise aus einem offenporigen Material, insbesondere aus einem Schaum-Kunststoff. Als äußere Wundabdeckung wird vorzugsweise eine Kunststofffolie verwendet, die über die Wunde gelegt und an den Wundrändern festgeklebt wird. Eine solche Folie als Wundabdeckung hat den Vorteil, die Wunde luftdicht abzuschließen, wodurch auch eine Belästigung durch die starke Geruchsbildung nekrotischer Wunden verhindert wird.

Besteht die Einlage aus einem offenporigen Schaumstoff, so kann die Einlage überschüssiges Wundsekret aufnehmen und speichern. Bei der Verwendung von Beuteln mit lebenden Maden dient die offenporige Einlage auch als Speicher für Luft und Feuchtigkeit, die für das Überleben der Maden notwendig sind, wenn die Wunde durch eine luftdichte Folie abgeschlossen ist.

Werden lebende Maden verwendet, so ist es vorteilhaft, wenn die luftdichte Abdeckung der Wunde durch eine Folie mit einem Belüftungssystem kombiniert wird. Über ein solches Belüftungssystem kann ausreichend Luft unter die Folie und in die offenporige Einlage gebracht werden, um das Überleben der Maden sicherzustellen. Um das Entweichen der in der Wunde entstehenden Gerüche über das Belüftungssystem zu verhindern, ist das Belüftungssystem zweckmäßigerweise verschließbar. Bei Bedarf von Luftzufuhr unter die luftdichte Abdeckung wird das Belüftungssystem geöffnet und eine Durchlüftung der offenporigen Einlage durchgeführt. Die austretende Gerüche können dabei erforderlichenfalls durch ein Geruchsfilter aufgefangen werden. Zwischen den einzelnen Belüftungsphasen kann das Belüftungssystem durch einen Deckel luftdicht verschlossen werden.

Im folgenden wird die Erfindung anhand der beigefügten Zeichnung erläutert. Es zeigen:
- Fig. 1: einen Vertikalschnitt durch eine Wunde mit dem Verbandsmaterial,
- Fig. 2: einen Fliegenlarven einschließenden Beutel und
- Fig.3: einen rasterförmig in Kammern unterteilten Beutel.

In eine Wunde 10 wird ein Beutel 12 eingelegt, der aus einem feinmaschigen netzartigen Material besteht. Der flächige Beutel 12 ist rigsum geschlossen. In den Beutel 12 sind steril gezüchtete lebende Maden 14 der Fliegengattung Lucilia sericata eingeschlossen. Auf den Beutel 12 wird eine entsprechen der Wundfläche zugeschnittene Einlage 16 aufgelegt, die aus einem offenporigen Schaumkunststoff besteht. Die gesamte Wunde 10 mit der durch den Beutel 12 gebildeten Wundauflage und der offenporigen Einlage 16 wird durch eine luftundurchlässige Folie 18 abgedeckt, die rings um die Wunde 10 mittels einer Klebeverbindung 20 luftdicht auf den Wundrändern festgeklebt wird. Die als Wundabdeckung dienende Folie 18 weist zur Belüftung einen Stutzen 22 auf, der durch einen mittels einer biegsamen Lasche mit dem Stutzen 22 verbundenen Deckel 24 verschließbar ist.

Zum Belüften der offenporigen Einlage 16 wird an den Stutzen 22 ein Belüftungssystem ggf. mit einem Geruchsfilter angeschlossen. Zwischen den einzelnen Belüftungen wird der Stutzen 22 mittels des Deckels 24 luftdicht verschlossen. In einfacher Weise kann die Belüftung auch durch Anheben und Komprimieren der Folie 18 manuell erfolgen.

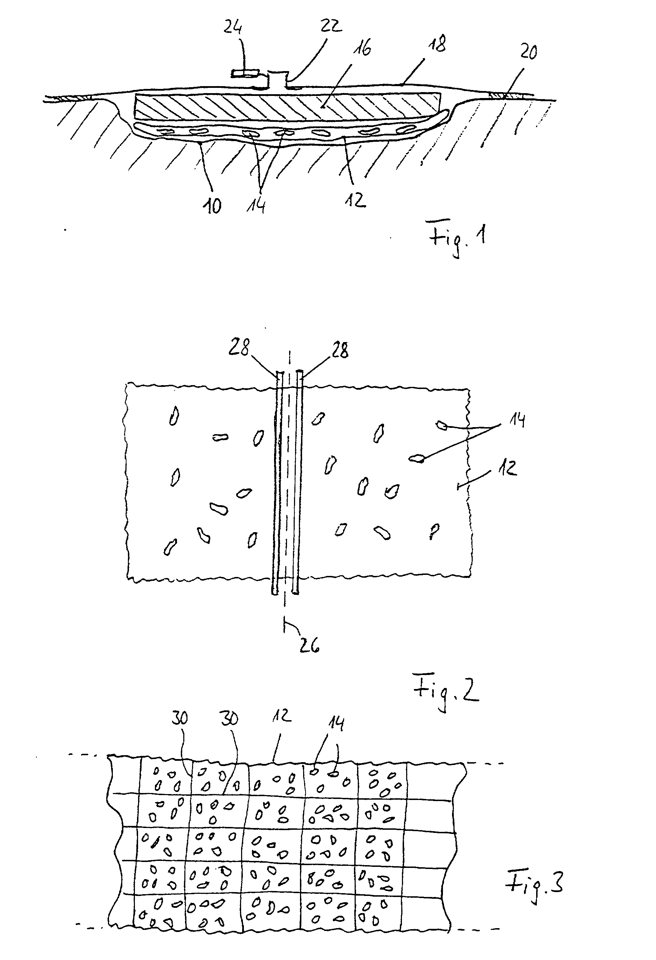

Vorzugsweise wird die Wundauflage in Form eines großflächigen Beutels 12 zur Verfügung gestellt. Dieser Beutel 12 kann bei Bedarf in kleinere Beutel unterteilt werden. Wie Fig. 2 zeigt, wird hierzu der Beutel beiderseits einer gewünschten Trennlinie 26 abgeschlossen, wozu beispielsweise Kunststoffklammern 28 verwendet werden können. Ebenso ist ein Verkleben der beiden Beutelflächen möglich. Nach dem Verschließen des Beutels durch die Klammern 28 bzw. nach dem Verkleben kann der Beutel 12 entlang der Trennlinie 26 durchgeschnitten werden, so daß zwei geschlossene Teilbeutel erhalten werden.

Anstelle eines lebende Maden 14 einschließenden Beutels 12 kann auch eine Wundauflage verwendet werden, die aus einem mit dem Sekret der Fliegenlarven 14 getränkten Material besteht.

In Fig. 3 ist eine weitere Ausführung der Wundauflage gezeigt. Der Beutel 12 besteht aus einer zweilagigen Bahn des feinmaschigen netzartigen Textilmaterials. In Längsrichtung und in Querrichtung der Bahnen verlaufende Schweiß- oder Klebenähte 30 verbinden die Bahnen jeweils, so daß der Beutel 12 in ein schachbrettartiges Raster von geschlossenen Kammern unterteilt ist. Jede dieser Kammern nimmt einige Fliegenlarven 14 auf. Diese können je nach Größe der Kammern 1 bis 5 oder 10 oder mehr Fliegenlarven 14 sein.

Die Unterteilung des Beutels 12 in einzelne Kammern hat den Vorteil, daß die Fliegenlarven 14 über die gesamte Fläche gleichmäßig verteilt gehalten werden. Weiter läßt die Unterteilung des Beutels 12 in ein Raster von Kammern in einfacher Weise ein den Abmessungen der Wunde bedarfsgerecht angepaßtes Zuschneiden der Wundauflage zu. Die Wundauflage kann in beliebiger Form zugeschnitten werden, wobei nur die in den jeweils durchschnittenen Kammern enthaltenen Maden 14 über die offene Schnittlinie verlorengehen.

## Patentansprüche

1. Verbandsmaterial zur Behandlung von Wunden mit dem Sekret von Fliegenlarven, insbesondere der Fliegengattung Lucilia, **gekennzeichnet durch** eine Wundauflage, die einen die Fliegenlarven (14) einschließenden Beutel (12) aus einem feinmaschigen netzartigen Material aufweist, das die Fliegenlarven (14) nicht aus dem Beutel entweichen läßt und dessen Porengröße einen ungehinderten Flüssigkeitsaustausch des Sekrets der Fliegenlarven (14) und des aufgelösten und verflüssigten nekrotischen Gewebes der Wunde ermöglicht.

2. Verbandsmaterial nach Anspruch 1,
**dadurch gekennzeichnet, dass** der Beutel (12) unterteilbar ist.

3. Verbandsmaterial nach Anspruch 2,
**dadurch gekennzeichnet, dass** der Beutel (12) in eine Vielzahl von rasterförmig angeordneten Kammern unterteilt ist, wobei jede Kammer einige Fliegenlarven (14) aufnimmt.

4. Verbandsmaterial nach einem der Ansprüche 1 bis 3,
**dadurch gekennzeichnet, dass** der Beutel (12) ein Futtermittel für die Fliegenlarven (14) enthält oder mit einem solchen Futtermittel imprägniert ist.

5. Verbandsmaterial nach einem der vorhergehenden Ansprüche,
**gekennzeichnet durch** eine auf der Wundauflage (12) angeordnete Einlage (16).

6. Verbandsmaterial nach Anspruch 5,
**dadurch gekennzeichnet, dass** die Einlage (16) aus einem offenporigen Schaumstoff, insbesondere einem Schaumkunststoff besteht.

7. Verbandsmaterial nach einem der vorhergehenden Ansprüche,
**gekennzeichnet durch** eine die Wundauflage (12) und ggf. die Einlage (16) abdeckende Wundabdeckung (18).

8. Verbandsmaterial nach Anspruch 7,
**dadurch gekennzeichnet, dass** die Wundabdeckung eine luftundurchlässige Folie (18), insbesondere eine Kunststofffolie ist.

9. Verbandsmaterial nach Anspruch 7 oder 8,
**dadurch gekennzeichnet, dass** die Wundabdeckung (18) eine Klebeverbindung (20) zum Aufkleben auf die Wundränder aufweist.

10. Verbandsmaterial nach Anspruch 8
**dadurch gekennzeichnet, dass** die luftundurchlässige Wundabdeckung (18) ein verschließbares Belüftungssystem (22, 24) aufweist.

## Claims

1. Bandage material for the treatment of wounds with the secretion of fly larvae, in particular the fly genus lucilia, **characterised by** a wound pad, which has a pouch (12) enclosing the fly larvae (14) made from a fine mesh net-type material, which does not allow the fly larvae (14) to escape from the pouch and the pore size of which allows an unobstructed exchange of fluid of the secretion of the fly larvae (14) and the dissolved and liquefied necrotic tissue of the wound.

2. Bandage material according to Claim 1, **characterised in that** the pouch (12) can be subdivided.

3. Bandage material according to Claim 2, **characterised in that** the pouch (12) is subdivided into a large number of chambers arranged in a grid shape, each chamber taking a few fly larvae (14).

4. Bandage material according to one of Claims 1 to 3, **characterised in that** the pouch (12) contains a feeding medium for the fly larvae (14) or is impregnated with such a feeding medium.

5. Bandage material according to one of the preceding claims, **characterised by** an insert (16) arranged on the wound pad (12).

6. Bandage material according to Claim 5, **characterised in that** the insert (16) consists of an open-pored foam material, in particular a foam plastic.

7. Bandage material according to one of the preceding claims, **characterised by** a wound covering (18) covering the wound pad (12) and possibly the insert (16).

8. Bandage material according to Claim 7, **characterised in that** the wound covering is an airtight foil (18), in particular a plastic foil.

9. Bandage material according to Claim 7 or 8, **characterised in that** the wound covering (18) has an adhesive connection (20) for sticking onto the edges of the wound.

10. Bandage material according to Claim 8, **characterised in that** the airtight wound covering (18) has a sealable ventilation system (22, 24).

## Revendications

1. Matériau de bandage pour le traitement de plaies avec la sécrétion de larves de mouches, en particulier du genre de mouches Lucilia,
**caractérisé par**
un revêtement de plaie qui possède un sachet (12) incluant les larves de mouche (14), en matériau du type réticulé à fines mailles qui ne laisse pas les larves de mouche (14) s'échapper du sachet et dont les tailles de pores rendent possible un échange de liquide non empêché de la sécrétion des larves de mouche (14) et du tissu nécrotique rendu liquide, de la plaie.

2. Matériau de bandage selon la revendication 1,
**caractérisé en ce que**
le sachet (12) peut être subdivisé.

3. Matériau de bandage selon la revendication 2,
**caractérisé en ce que**
le sachet (12) est subdivisé en une multiplicité de chambres, disposées sous forme de réseau, chaque cavité recevant quelques larves de mouche (14).

4. Matériau de bandage selon l'une des revendications 1 à 3,
**caractérisé en ce que**
le sachet (12) contient un aliment pour les larves de mouche (14) ou est imprégné d'un tel aliment.

5. Matériau de bandage selon l'une des revendications précédentes,
**caractérisé par**
une insertion (16) disposée sur le revêtement de plaie (12).

6. Matériau de bandage selon la revendication 5,
**caractérisé en ce que**
l'insertion (16) consiste en une mousse à pores ouverts, en particulier une matière plastique en mousse.

7. Matériau de bandage selon l'une des revendications précédentes,
**caractérisé par**
un revêtement de plaie (12) et le cas échéant le recouvrement de plaie recouvrant l'insertion (16).

8. Matériau de bandage selon la revendication 7,
**caractérisé en ce que**
le revêtement de plaie est une feuille imperméable à l'air (18), en particulier une feuille de matière plastique.

9. Matériau de bandage selon la revendication 7 ou la revendication 8,
**caractérisé en ce que**
le revêtement de plaie (18) possède une liaison de colle (20) en vue du collage sur les bords de la plaie.

10. Matériau de bandage selon la revendication 8,
**caractérisé en ce que**
le recouvrement de plaie, imperméable à l'air, possède un système d'aération obturable (22, 24).
